# EUROPÄISCHE PATENTSCHRIFT

(11) **EP 1 565 160 B1**
(45) Veröffentlichungstag und Bekanntmachung des Hinweises auf die Patenterteilung: **22.01.2014**
(21) Anmeldenummer: 03779793.3
(22) Anmeldetag: 26.09.2003
(51) Int. Cl.: A61K 9/00

(54) **DARREICHUNGSFORM FÜR PHARMAZEUTISCH AKTIVE PEPTIDE MIT ANHALTENDER WIRKSTOFFFREIGABE UND VERFAHREN ZU DEREN HERSTELLUNG**
ADMINISTRATION FORM FOR PHARMACEUTICALLY ACTIVE PEPTIDES WITH SUSTAINED RELEASE AND METHOD FOR THE PRODUCTION THEREOF
FORME D'ADMINISTRATION DE PEPTIDES PHARMACEUTIQUEMENT ACTIFS AVEC LIBERATION PROLONGEE DU PRINCIPE ACTIF ET SON PROCEDE DE PRODUCTION

(30) Priorität: 27.09.2002 DE 10245525; 27.09.2002 US 414225 P; 26.04.2003 DE 10320051
(43) Veröffentlichungstag der Anmeldung: 24.08.2005
(73) Patentinhaber: Æterna Zentaris GmbH, 60314 Frankfurt am Main (DE)
(72) Erfinder: BAUER, Horst, 91217 Hersbruck (DE); REISSMANN, Thomas, 60437 Frankfurt am Main (DE); ROMEIS, Peter, 63571 Gelnhausen (DE); ROESSLER, Berthold, 33790 Halle (DE)
(74) Vertreter: Potter Clarkson LLP
(86) Internationale Anmeldenummer: PCT/EP2003/010732
(87) Internationale Veröffentlichungsnummer: WO 2004/030650

(56) Entgegenhaltungen:
- WO-A-99/48517
- US-A- 5 411 951
- US-A- 5 595 760
- US-A- 5 916 582
- JIANG G ET AL: "BETIDAMINO ACID-SCAN OF THE GNRH ANTAGONIST ACYLINE" JOURNAL OF MEDICINAL CHEMISTRY, AMERICAN CHEMICAL SOCIETY. WASHINGTON, US, Bd. 40, 1997, Seiten 3739-3748, XP002071836 ISSN: 0022-2623

## Beschreibung

### Gebiet der Erfindung

Die Erfindung betrifft pharmazeutische Darreichungsformen mit anhaltender Wirkstofffreigabe (sustained release) mit mindestens einem pharmakologisch aktiven Peptid, ein Verfahren zu deren Herstellung, einen Kit umfassend ein lyophilisiertes Peptid und eine wässrige Lösung eines anorganischen Salzes oder Essigsäure-Salzes und die Verwendung einer wässrigen Lösung eines anorganischen oder Essigsäure-Salzes zur Herstellung einer pharmazeutischen Darreichungsform, die über einen längeren Zeitraum eine anhaltende Peptidfreisetzung aufweist.

### Beschreibung des Standes der Technik

Im Stand der Technik sind die folgenden pharmazeutischen Darreichungsformen mit anhaltender Freigabe eines pharmazeutisch aktiven Peptids bekannt:
1. Pharmazeutische Darreichungsformen mit mikroverkapselten und/oder eingebetteten und/oder konjugierten pharmazeutisch aktiven Peptiden in einer biologisch abbaubaren polymerischen Matrix (z. B. beschrieben in: Maulding, H. V., J. Controlled Release (1987), 6, 167-76; Siegel, R. A., Langer, R. Pharm.Res. (1984). 1,2-10; Patent WO 9832423, Patent WO 2001078687).
2. Pharmazeutische Darreichungsformen umfassend aus kaum wasserlöslichen Komplexen des pharmazeutisch aktiven Peptids und einem organischen Trägermolekül, wie z.B. Polysacchariden. (z.B. beschrieben in: Patent WO 2000047234).

In beiden Fällen führt der enzymatische Abbau von Matrix oder Komplex zur anhaltenden Freigabe des Peptids.

### Probleme im Zusammenhang mit dem Stand der Technik

Zur Herstellung der bekannten Mikrokapseln oder Partikel und unlöslichen Komplexe der Peptidverbindungen sind hoch anspruchsvolle Verfahrensweisen notwendig, um Darreichungsformen mit anhaltender Wirkstofffreigabe zu erhalten. Normalerweise entstehen unlösliche oder schwerlösliche Verbindungen durch Ausfällung der Peptidverbindung mit dem Gegenion. Der Niederschlag wird durch Filtration und Zentrifugation aufgefangen, mit Wasser gewaschen und getrocknet. In den meisten Fällen wird das feste Material dann pulverisiert. Alle einzelnen Verfahrensschritte der Herstellung müssen unter GMP Bedingungen in einem aseptischen Arbeitsbereich durchgeführt werden, damit auf diese Weise die Sterilität des Endproduktes garantiert werden kann.

Bei dem Herstellungsverfahrensweisen von Mikrokapseln werden mehr oder weniger toxische organische Lösungsmittel verwendet, um die biologisch abbaubare Polymermatrix zu lösen. Anschliessend werden die gelöste aktive Substanz und die Polymere der Matrix emulgiert. Nach Verdampfung des organischen Lösungsmittels werden die Partikel oder die Mikrokapseln getrennt, gewaschen und getrocknet.

### Beschreibung der Erfindung

Überraschenderweise wurde nun gefunden, dass man Darreichungsformen mit anhaltender Wirkstofffreigabe (sustained release) für pharmazeutisch aktive Peptide durch Rekonstitution einer lyophilisierten Peptidverbindung mit einer niedrigkonzentrierten anorganischen Salzlösung vor der Verabreichung erhält, wobei die Menge an lyophilisierter Peptidverbindung so gewählt wird, dass nach der Rekonstitution eine hochkonzentrierte Peptidlösung bzw. suspension vorliegt.

Als eine mögliche Erklärung wird vermutet, dass es unter diesen Bedingungen zur kontrollierten Entwicklung von Aggregaten der Peptidverbindungen kommt, dessen bzw. deren Auflösung verzögert ist. Die Folge ist dann die gefundene anhaltende Freigabe dieses Wirkstoffes in den Kreislauf. Dabei führt die Ausbildung der Aggregate zu einer kolloidalen Dispersion, deren Viskosität durch die Konzentration der Peptidverbindung, der Salzkonzentration und der Standzeit nach Rekonstitution beeinflusst werden.

Gemäß der vorliegenden Erfindung werden pharmazeutische Gel-Zubereitungen umfassend mindestens eine pharmazeutisch aktive ionische Peptidverbindung in einer vorbestimmten Menge des Wertes Xₒₚₜᵢₘᵤₘ (in mg Peptid pro ml der Zubereitung) vermischt mit einer wässrigen Lösung eines anorganischen oder Essigsäure-Salzes in einer vorbestimmten Konzentration des Wertes Yₒₚₜᵢₘᵤₘ (in % Gewicht/Volumen), wobei nach dem Vermischen die Verabreichung sofort erfolgen oder eine Standzeit von bis zu etwa 120 Minuten eingehalten werden kann und wobei der Wert Xₒₚₜᵢₘᵤₘ ausgewählt werden kann durch eine Testmethode A, umfassend die Stufen Verabreichen von verschiedenen Mengen Xₙ (Anzahl der verschiedenen Mengen n, wobei n ≥ 1) (in mg) des Peptids als ein Gemisch mit einer isotonischen wäßrigen Lösung von Mannitol an bzw. zu einem Testsystem und Auswählen der Menge Xₒₚₜᵢₘᵤₘ (in mg Peptid pro ml Mischung), die im Versuch die günstigsten Blutplasmaspiegel des Peptids in dem Testsystem in Hinblick auf Cₘₐₓ (maximale Blutplasmakonzentration) und tₘₐₓ (Zeitdauer zum Erreichen von Cₘₐₓ) lieferte, und wobei die Konzentration Yₒₚₜᵢₘᵤₘ ausgewählt werden durch eine Testmethode B umfassend die Stufen Verabreichen der Menge Xₒₚₜᵢₘᵤₘ (in mg Peptid pro ml Mischung) des Peptids als ein Gemisch mit wäßrigen Lösungen, welche sich in der Konzentration Yₙ (Anzahl der verschiedenen Konzentrationen n, wobei n ≥ 1) (in % Gewicht/Volumen) unterschieden, an bzw. zu einem Testsystem und Auswählen der Konzentration Yₒₚₜᵢₘᵤₘ (in % Gewicht/Volumen) wurde festgelegt als diejenige Konzentration, die im Versuch den höchsten Wert für die Plasmakonzentration C_{active} ergab, wobei Cₘᵢₙ < C_{active} > Cₘᵢₙ (Cₘᵢₙ = niedrigste Plasmakonzentration des Peptids bei der das Peptid im Versuch noch eine ausreichende pharmazeutische Wirkung hat). Gleichzeitig hat es einen Einfluss auf den Zeitraum t_{activa} bis die höchste Konzentration im Plasma erreicht wird, wobei t_{active} > tₘₐₓ, bereitgestellt.

Gemäß einer weiteren Ausführungsform wird eine pharmazeutische Zubereitung bereitgestellt, dadurch charakterisiert, dass die pharmazeutisch aktive ionische Peptidverbindung kationisch ist.

Gemäß einer weiteren Ausführungsform wird eine pharmazeutische Zubereitung bereitgestellt, dadurch charakterisiert, dass die pharmazeutisch aktive ionische Peptidverbindung anionisch ist.

Gemäß einer weiteren Ausführungsform wird eine pharmazeutische Zubereitung bereitgestellt, dadurch charakterisiert, dass die pharmazeutisch aktive ionische Peptidverbindung ein mono-, di- oder multivalentes kationisches oder anionisches Peptid ist.

Gemäß einer weiteren Ausführungsform wird eine pharmazeutische Zubereitung bereitgestellt, dadurch charakterisiert, dass die pharmazeutisch aktive ionische Peptidverbindung ein mono-, di- oder multivalentes ampholytisches Peptid ist.

Gemäß einer weiteren Ausführungsform wird eine pharmazeutische Zubereitung bereitgestellt, dadurch charakterisiert, dass die pharmazeutisch aktive, ionische Peptidverbindung eine Länge von 5 bis 20 Aminosäuren aufweist.

Gemäß einer weiteren Ausführungsform wird eine pharmazeutische Zubereitung bereitgestellt, dadurch charakterisiert, dass die pharmazeutisch aktive, ionische Peptidverbindung eine Länge von 8 bis 12 Aminosäuren aufweist.

Gemäß einer weiteren Ausführungsform wird eine pharmazeutische Zubereitung bereitgestellt, dadurch charakterisiert, dass die pharmazeutisch aktive, ionische Peptidverbindung ein GnRH-Analogon ist.

Gemäß einer weiteren Ausführungsform wird eine pharmazeutische Zubereitung bereitgestellt, dadurch charakterisiert, dass die pharmazeutisch aktive, ionische Peptidverbindung ein GnRH-Antagonist ist.

Gemäß einer weiteren Ausführungsform wird eine pharmazeutische Zubereitung bereitgestellt, dadurch charakterisiert, dass die pharmazeutisch aktive, ionische Peptidverbindung aus der Gruppe bestehend aus Cetrorelix, Teverelix, Abarelix, Ganirelix, Azaline B, Antide, Detirelix, Ramorelix, Degarelix, D-63153 oder deren pharmazeutisch aktives Salz oder Gemischen davon ausgewählt worden ist. Gemäß einer weiteren Ausführungsform wird eine pharmazeutische Zubereitung bereitgestellt, dadurch charakterisiert, dass die pharmazeutisch aktive, ionische Peptidverbindung der GnRH-Antagonist D-63153 ist.

Gemäß einer weiteren Ausführungsform wird eine pharmazeutische Zubereitung bereitgestellt, dadurch charakterisiert, dass es sich bei dem anorganischen Salz oder dem Essigsäuresatz um ein physiologisch verträgliches Salz handelt.

Gemäß einer weiteren Ausführungsform wird eine pharmazeutische Zubereitung bereitgestellt, dadurch charakterisiert, dass das wässrige anorganische Salz oder Essigsäuresalz aus der Gruppe bestehend aus Natriumchlorid, Kalziumchlorid, Magnesiumchlorid, Natriumacetat, Kalziumacetat und Magnesiumacetat ausgewählt worden ist.

Gemäß einer weiteren Ausführungsform wird eine pharmazeutische Zubereitung bereitgestellt, dadurch charakterisiert, dass die Mischung der pharmazeutisch aktiven, ionischen Peptidverbindung und der wässrigen Lösung des anorganischen Salzes oder des Essigsäuresalzes eine flüssige Suspension oder eine halbfeste Dispersion ist.

Gemäß einer weiteren Ausführungsform wird eine pharmazeutische Zubereitung bereitgestellt, dadurch charakterisiert, dass die Menge X der pharmazeutisch aktiven, ionischen Peptidverbindung im Bereich von etwa 5 bis etwa 50 mg pro ml der Gesamtmenge der pharmazeutischen Zubereitung liegt.

Gemäß einer weiteren Ausführungsform wird eine pharmazeutische Zubereitung bereitgestellt, dadurch charakterisiert, dass die Menge X der pharmazeutisch aktiven, ionischen Peptidverbindung im Bereich von etwa 10 bis etwa 50 mg pro ml der Gesamtmenge der pharmazeutischen Zubereitung liegt.

Gemäß einer weiteren Ausführungsform wird eine pharmazeutische Zubereitung bereitgestellt, dadurch charakterisiert, dass die Menge X der pharmazeutisch aktiven, ionischen Peptidverbindung im Bereich von etwa 20 bis etwa 30 mg pro ml der Gesamtmenge der pharmazeutischen Zubereitung liegt.

Gemäß einer weiteren Ausführungsform wird eine pharmazeutische Zubereitung bereitgestellt, dadurch charakterisiert, dass die Menge X der pharmazeutisch aktiven, ionischen Peptidverbindung im Bereich von etwa 25 mg pro ml der Gesamtmenge der pharmazeutischen Zubereitung liegt.

Gemäß einer weiteren Ausführungsform wird eine pharmazeutische Zubereitung bereitgestellt, dadurch charakterisiert, dass D-63153 die pharmazeutisch aktive, ionische Peptidverbindung ist und die Menge X im Bereich von etwa 5 bis etwa 50 mg pro ml der Gesamtmenge der pharmazeutischen Zubereitung liegt.

Gemäß einer weiteren Ausführungsform wird eine pharmazeutische Zubereitung bereitgestellt, dadurch charakterisiert, dass D-63153 die pharmazeutisch aktive, ionische Peptidverbindung ist und die Menge X im Bereich von etwa 10 bis etwa 50 mg pro ml der Gesamtmenge der pharmazeutischen Zubereitung liegt.

Gemäß einer weiteren Ausführungsform wird eine pharmazeutische Zubereitung bereitgestellt, dadurch charakterisiert, dass D-63153 die pharmazeutisch aktive, ionische Peptidverbindung ist und die Menge X im Bereich von etwa 20 bis etwa 30 mg pro ml der Gesamtmenge der pharmazeutischen Zubereitung liegt

Gemäß einer weiteren Ausführungsform wird eine pharmazeutische Zubereitung bereitgestellt, dadurch charakterisiert, dass D-63153 die pharmazeutisch aktive, ionische Peptidverbindung ist und die Menge X im Bereich von etwa 25 mg pro ml der Gesamtmenge der pharmazeutischen Zubereitung liegt.

Gemäß einer weiteren Ausführungsform wird eine pharmazeutische Zubereitung bereitgestellt, dadurch charakterisiert, dass die Konzentration Y der wässrigen anorganischen oder Essigsäuresalziösung gleich oder niedriger als 0.9% (Gewicht/Volumen) ist.

Gemäß einer weiteren Ausführungsform wird eine pharmazeutische Zubereitung bereitgestellt, dadurch charakterisiert, dass die Konzentration Y der wässrigen anorganischen oder Essigsäuresalz-Lösung im Bereich von etwa 0.01% bis etwa 0.9% (Gewicht/Volumen) liegt.

Gemäß einer weiteren Ausführungsform wird eine pharmazeutische Zubereitung bereitgestellt, dadurch charakterisiert, dass die Konzentration Y der wässrigen anorganischen oder Essigsäuresalz-Lösung im Bereich von etwa 0.05% bis etwa 0.5% (Gewicht/Volumen) liegt.

Gemäß einer weiteren Ausführungsform wird eine pharmazeutische Zubereitung bereitgestellt, dadurch charakterisiert, dass die Konzentration Y der wässrigen anorganischen oder Essigsäuresatz-Lösung etwa 0.1 % (Gewicht/Volumen) ist.

Gemäß einer weiteren Ausführungsform wird eine pharmazeutische Zubereitung bereitgestellt, dadurch charakterisiert, dass das anorganische Salz Natriumchlorid ist und dass die Konzentration Y gleich oder niedriger als etwa 0.9 % (Gewicht/Volumen) ist.

Gemäß einer weiteren Ausführungsform wird eine pharmazeutische Zubereitung bereitgestellt, dadurch charakterisiert, dass das anorganische Salz Natriumchlorid ist und dass die Konzentration Y im Bereich von etwa 0.01% bis etwa 0.9% (Gewicht/Volumen) liegt.

Gemäß einer weiteren Ausführungsform wird eine pharmazeutische Zubereitung bereitgestellt, dadurch charakterisiert, dass das anorganische Salz Natriumchlorid ist und dass die Konzentration Y im Bereich von etwa 0.05% bis etwa 0.5% (Gewicht/Volumen) liegt.

Gemäß einer weiteren Ausführungsform wird eine pharmazeutische Zubereitung bereitgestellt, dadurch charakterisiert, dass das anorganische Salz Natriumchlorid ist und das die Konzentration Y etwa 0.1% (Gewicht/Volumen) beträgt.

Gemäß einer weiteren Ausführungsform wird eine pharmazeutische Zubereitung bereitgestellt, dadurch charakterisiert, dass zumindest eine der pharmazeutisch aktiven ionischen Peptidverbindung D-63153 ist und das anorganische Salz Natriumchlorid ist.

Gemäß einer weiteren Ausführungsform wird eine pharmazeutische Zubereitung bereitgestellt, dadurch charakterisiert, dass zumindest eine der pharmazeutisch aktiven ionischen Peptidverbindung D-63153 ist und deren Menge X etwa 25 ml pro ml der Zubereitung beträgt und dass das anorganische Salz Natriumchlorid ist und dessen Konzentration Y etwa 0.1 % (Gewicht/ Volumen) beträgt.

Gemäß einem weiteren Aspekt der Erfindung wird ein Verfahren zur Herstellung einer pharmazeutischen Zubereitung bereitgestellt, umfassend die Schritte A) Zusammenbringen einer Menge Xₒₚₜᵢₘᵤₘ (in mg pro ml der fertigen Zubereitung) von mindestens einer pharmazeutisch aktiven Peptidverbindung in lyophilisierter Form und einer wässrigen Lösung eines anorganischen oder Essigsäure-Salzes in einer Konzentration mit dem Wert Yₒₚₜᵢₘᵤₘ (% Gewicht/Volumen) und A) Vermischen der Komponenten.

Gemäß einer weiteren Ausführungsform der Erfindung wird ein Verfahren zur Herstellung einer pharmazeutischen Zubereitung bereitgestellt, dadurch charakterisiert, dass die pharmazeutisch aktive, ionische Peptidverbindung D-63153 ist und das anorganische Salz Natriumchlorid ist.

Gemäß einer weiteren Ausführungsform der Erfindung wird ein Verfahren zur Herstellung einer pharmazeutischen Zubereitung bereitgestellt, dadurch charakterisiert, dass die pharmazeutisch aktive, ionische Peptidverbindung D-63153 ist und dessen Menge etwa 25 mg/ml beträgt und dass das anorganische Salz Natriumchlorid ist und dessen Konzentration etwa 0.1 % (Gewicht/Volumen) beträgt.

Gemäß einer weiteren Ausführungsform der Erfindung wird ein Verfahren zur Herstellung einer pharmazeutischen Zubereitung bereitgestellt, charakterisiert durch weiter umfassend den Schritt der Sterilisation der Peptidformulierung durch Gammastrahlen- oder Elektronenstrahlbestrahlung stattfindet.

Gemäß einer weiteren Ausführungsform der Erfindung wird ein Verfahren zur Herstellung einer pharmazeutischen Zubereitung bereitgestellt, dadurch charakterisiert, dass die Herstellung der Peptiformulierung unter Anwendung aseptischer Verfahrensweisen erfolgt.

Gemäß einem weiteren Aspekt der Erfindung wird ein Kit zur Herstellung einer pharmazeutischen Zubereitung bereitgestellt, umfassend eine vorher festgelegte Menge X (in mg pro ml der fertigen Zubereitung) einer pharmazeutisch aktiven, ionischen Peptidverbindung in lyophilisierter Form and einer wäßrigen Lösung eines anorganischen oder Essigsäure-Salzes in einer vorher festgelegten Konzentration Y % (Gewicht/Volumen).

Gemäß einer weiteren Ausführungsform der Erfindung wird ein Kit zur Herstellung einer pharmazeutischen Zubereitung bereitgestellt , dadurch charakterisiert, dass die pharmazeutisch aktive Peptidverbindung D-63153 in lyophilisierter Form ist.

Gemäß einer weiteren Ausführungsform der Erfindung wird ein Kit zur Herstellung einer pharmazeutischen Zubereitung bereitgestellt, dadurch charakterisiert, dass das D-63153 Lyophilisat zusätzlich Mannit enthält.

Gemäß einer weiteren Ausführungsform der Erfindung wird ein Kit zur Herstellung einer pharmazeutischen Zubereitung bereitgestellt, dadurch charakterisiert, dass das anorganische Salz Natriumchlorid ist

Gemäß einer weiteren Ausführungsform der Erfindung wird ein Kit zur Herstellung einer pharmazeutischen Zubereitung bereitgestellt, dadurch charakterisiert, dass die Menge X an D-63153 etwa 25 mg pro fertiger Zubereitung und die Konzentration der wäßrigen Natriumchloridlösung etwa 0.1 % Gewicht/Volumen beträgt.

Gemäß einem weiteren Aspekt der Erfindung wird ein Verfahren zur Behandlung eines Patienten mit einer pharmazeutisch aktiven Peptidverbindung bereitgestellt, dadurch charakterisiert, dass eine pharmazeutische Zubereitung gemäß einem der vorstehend genannten Ansprüche subkutan oder intramuskulär dem Patienten mittels einer Spritze verabreicht wird.

Gemäß einer weiteren Ausführungsform der Erfindung wird ein Verfahren zur Behandlung eines Patienten mit einer pharmazeutisch aktiven Peptidverbindung bereitgestellt, dadurch charakterisiert, dass die verabreichte pharmazeutische Zubereitung eine anhaltende pharmazeutische Aktivität aufweist.

Gemäß einer weiteren Ausführungsform der Erfindung wird ein Verfahren zur Behandlung eines Patienten mit einer pharmazeutisch aktiven Peptidverbindung bereitgestellt, dadurch charakterisiert, dass die verabreichte pharmazeutische Zubereitung eine anhaltende pharmazeutische Aktivität während mindestens 4 Wochen aufweist.

Gemäß einer weiteren Ausführungsform der Erfindung wird ein Verfahren zur Behandlung eines Patienten mit einer pharmazeutisch aktiven Peptidverbindung bereitgestellt, dadurch charakterisiert, dass die verabreichte pharmazeutische Zubereitung eine anhaltende pharmazeutische Aktivität während mindestens 8 Wochen aufweist.

Gemäß einer weiteren Ausführungsform der Erfindung wird ein Verfahren zur Behandlung eines Patienten mit einer pharmazeutisch aktiven Peptidverbindung bereitgestellt, dadurch charakterisiert, dass die verabreichte pharmazeutische Zubereitung eine anhaltende pharmazeutische Aktivität während mindestens 12 Wochen aufweist.

Gemäß einer weiteren Ausführungsform der Erfindung wird ein Verfahren zur Behandlung einer hormonabhängigen Erkrankung an einem Patienten durch subkutane oder intramuskuläre Verabreichung der vorstehend genannten pharmazeutischen Zubereitungen bei einem Patienten, der dies benötigt, bereitgestellt.

Gemäß einem weiteren Aspekt der Erfindung wird ein Verfahren zur Behandlung von Prostatakrebs bei einem Patienten durch subkutane oder intramuskuläre Verabreichung der vorstehend beschriebenen erfindungsgemässen pharmazeutischen Zubereitung an einem Patienten, der dies benötigt, bereitgestellt.

Gemäß einem weiteren Aspekt der Erfindung wird ein Verfahren zur Behandlung von Brustkrebs bei einem Patienten durch subkutane oder intramuskuläre Verabreichung der vorstehend beschriebenen efindungsgemässen pharmazeutischen Zubereitung an einem Patienten, der dies benötigt, bereitgestellt.

Gemäß einem weiteren Aspekt der Erfindung wird ein Verfahren zur Behandlung von Uterusmyomen bei einem Patienten durch subkutane oder intramuskuläre Verabreichung der vorstehend beschriebenen erfindungsgemässen pharmazeutischen Zubereitung an einem Patienten, der dies benötigt, bereitgestellt.

Gemäß einem weiteren Aspekt der Erfindung wird ein Verfahren zur Behandlung von Endometriose bei einem Patienten durch subkutane oder intramuskuläre Verabreichung der vorstehend beschriebenen erfindungsgemässen pharmazeutischen Zubereitung an einem Patienten, der dies benötigt, bereitgestellt.

Gemäß einem weiteren Aspekt der Erfindung wird ein Verfahren zur Behandlung von Pubertas Precox bei einem Patienten durch subkutane oder intramuskuläre Verabreichung der vorstehend beschriebenen erfindungsgemässen pharmazeutischen Zubereitung an einem Patienten, der dies benötigt, bereitgestellt.

Gemäß einem weiteren Aspekt der Erfindung wird ein Verfahren zur Modifizierung der Fortpftanzungsfunktion bei einem Patienten durch subkutane oder intramuskuläre Verabreichung der vorstehend beschriebenen erfindungsgemässen pharmazeutischen Zubereitung an einem Patienten, der dies benötigt, bereitgestellt.

Gemäß einem weiteren Aspekt der Erfindung wird eine pharmazeutische Zubereitung bereitgestellt, dadurch charakterisiert, dass die Mischung der pharmazeutisch aktiven, ionischen Peptidverbindung und der wässrigen Lösung des anorganischen Salzes oder des Essigsäuresalzes eine molekular- oder kolloidaldisperse Mischung ist, die von flüssiger bis zu halbfester Konsistenz sein kann.

Gemäß einem weiteren Aspekt der Erfindung wird eine pharmazeutische Zubereitung bereitgestellt, dadurch charakterisiert, dass durch Rekonstitution eine kolloidale Dispersion entsteht.

Gemäß einem weiteren Aspekt der Erfindung wird eine pharmazeutische Zubereitung bereitgestellt, dadurch charakterisiert, dass durch Lagerung bzw. Stehenlassen nach Rekonstitution eine kolloidale Dispersion entsteht, die in Abhängigkeit von der Zeit ihre Viskosität verändert und damit die Reproduzierbarkeit der verzögerten Wirkstofffreisetzung verbessert.

Gemäß einem weiteren Aspekt der Erfindung wird ein, Kit umfassend ein lyophilisiertes pharmazeutisch aktives Peptid, beispielsweise D-63153, gegebenenfalls zusammen mit einem oder mehreren pharmazeutisch verträglichen Hilfs- oder Zusatzstoffen, und eine niedrigkonzentrierte wäßrige Lösung eines anorganischen Salzes, vorzugsweise Natriumchlorid, bereitgestellt.

In einer bevorzugten Ausführungsform ist die Peptidverbindung der Darreichungsform ein GnRH-Analoges, besser noch ein GnRH Antagonist, und das anorganische Salz ist ein hochlösliches physiologisches Salz, vorzugsweise Natriumchlorid.

Auf Grund der parenteralen Verabreichung ist es erforderlich, dass die pulverisierte Peptidverbindung und die Lösung für die Rekonstitution steril sind.

Die vorliegende Erfindung ermöglicht die einfache Herstellung von Suspensionen mit anhaltender Wirkstofffreigabe einer Peptidverbindung, bevorzugt eines GnRH-Antagonisten. Diese wird durch Rekonstitution eines hochkonzentrierten Lyophilisates der Peptidverbindung, enthaltend Mannitol, mit einer verdünnten anorganischen Salzlösung (z.B. Natriumchtoridlösung) erhalten.

Die Bildung der erfindungsgemäßen pharmazeutischen Formulierung ist dabei von folgenden Parametern abhängig:
1. der Konzentration der Peptidverbindung in der Lösung nach Rekonstitution
2. der Konzentration des anorganischen Salzes in dem zur Rekonstitution eingesetzten Lösungsmittels
3. der Standzeit der Lösung nach Rekonstitution und dem damit erhalten Ausmaß der Aggregation, die durch die Viskositätserhöhung wiedergespiegelt wird.

Die hohe Konzentration der Peptidverbindung führt zu dessen Aggregation, die durch Zugabe einer anorganischen Salzlösung kontrolliert werden kann. Mit zunehmender Salzkonzentration nimmt die Löslichkeit der Peptidverbindung ab. Die kolloidalen Eigenschaften treten gegenüber den Lösungseigenschaften in den Vordergrund, was durch die steigende Viskosität bis hin zum Gel verdeutlicht wird. Der Begriff "Gel" steht hierbei für ein bikohärentes System bestehend aus dem Peptidaggregat als der festen Phase und Wasser als der flüssigen Phase.

Die erfindungsgemäßen Darreichungsformen für pharmazeutisch aktive Peptide mit anhaltender Wirkstofffreigabe (sustained release) liegen vor der Verabreichung stets als Gel vor.

In einem idealen Bereich der Salzkonzentration, kombiniert mit einer geeigneten Menge an Peptidverbindungen, kann eine anhaltenden Wirkstofffreigabe über einen Zeitraum von 4 Wochen oder mehr erhalten werden.

Für die anorganische Salzlösung kann jedes physiologisch verträgliche (tolerierte) anorganische Salz verwendet werden, vorzugsweise Natriumchlorid.

Die Rekonstitution erfolgt mit einer niedrigkonzentrierten Salzlösung. Die Konzentration soll dabei gleich oder kleiner als etwa 0,9 % (Gewicht/Volumen) sein, vorzugsweise im Bereich von etwa 0,01 % bis etwa 0,9 %, besonders bevorzugt im Bereich von etwa 0,05 bis etwa 0,5 % (Gew./Vol.), ganz vorzugsweise bei etwa 0,1 % (Gew./Vol.) liegen.

Bevorzugt ist einen niedrigkonzentrierte Natriumchloridlösung mit eine Natriumchloridkonzentration im Bereich von etwa 0,05 bis etwa 0,5 % (Gew./Vol.), vorzugsweise von etwa 0,1 % (Gew./Vol.).

Das Peptid in der Formulierung ist eine pharmakologisch wirksame peptidische Verbindung welches ein mono-, di oder multivalentes kationisches oder anionisches Peptid sein kann. Das Peptid kann in der Länge aus 5 bis 20 Aminosäuren bestehen, mehr bevorzugt aus 8 bis 12 Aminosäuren in der Länge. Mehr im Detail ist die Peptidverbindung ein GnRH Analoges und der GnRH Analoge ist ein GnRH-Antagonist. GnRH Analoge sind z.B. Cetrorelix, Teverelix (Deghenghi et al., Biomed & Pharmacother 1993, 47, 107), Abarelix (Molineaux et al., Molecular Urology 1998, 2, 265), Ganirelix (Nestor et al., J. Med. Chem. 1992, 35,3942), Azaline B, Antide, A-75998 (Cannon et al., J. Pharm. Sci. 1995, 84, 953), Detirelix (Andreyko et al., J. Clin. Endocrinol. Metab. 1992, 74, 399), RS-68439 , Ramorelix (Stöckemann and Sandow, J. Cancer Res. Clin. Oncol. 1993, 119, 457), Degarelix (Broqua, P.; Riviere et al., JPET 301, 95), D-63153 (PCT: EP00/02165).

Die Strukturen der oben genannten GnRH-Analoge werden beispielsweise in den oben angegebenen Referenzen und in den nachfolgenden Übersichtsartikeln dargestellt: Behre et al., GnRH antagonists: an overview, Proceedings of the 2nd World Conference on Ovulation Induction, The Parthenon Publishing Group Ltd, UK; Kutscher et al., Angew. Chem. 1997,109,2240.

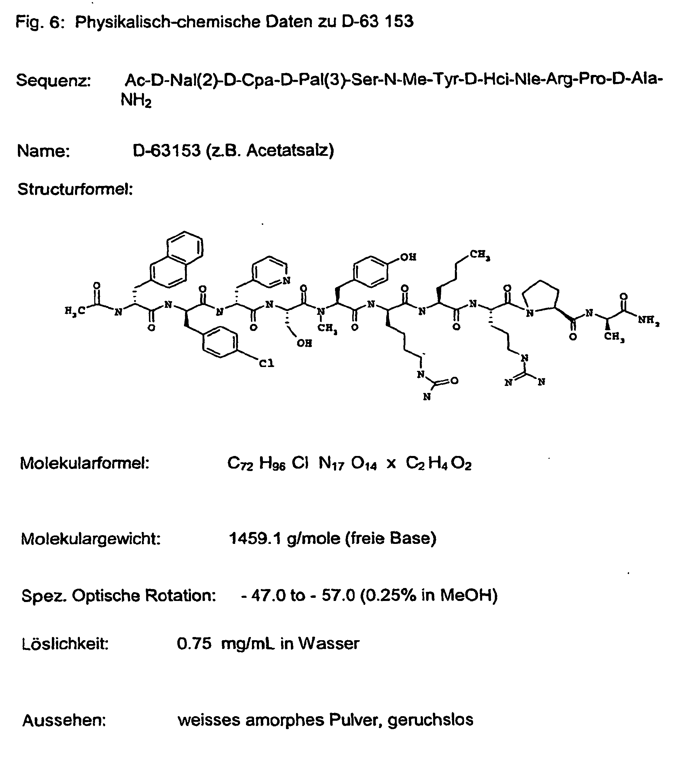

Die Verbindung D-63 153 ist unter anderem in der deutschen Patentanmeldung Nr. DE 199 11 771.3 beschrieben. Die phsikalisch-chemischen Daten sind in Fig. 6 zusammengefasst.

Die Konzentration des pharmazeutisch aktiven Peptids kann im Bereich von etwa 5 mg/ml bis etwa 50 mg/ml, vorzugsweise etwa 10 mg/ml bis etwa 50 mg/ml, besonders bevorzugt etwa 20 mg/ml bis etwa 30 mg/ml und ganz besonders bevorzugt bei etwa 25 mg/ml liegen (ml = Gesamtvolumen der fertigen Darreichungsform).

Alle pharmazeutisch aktiven Peptide können bei den genannten Konzentrationen eingesetzt werden. Das Peptid D-63 153 ist besonders bevorzugt.

Gemäß einem weiteren Aspekt der vorliegenden Erfindung wird ein Verfahren zur Herstellung von Darreichungsformen für pharmazeutisch aktive Peptide mit anhaltender Wirkstofffreigabe (sustained release) bereitgestellt.

Entsprechend der Erfindung wird das Acetatsalz Base der Peptidverbindung in wässriger Essigsäure vollständig gelöst bis eine klare Lösung vorliegt. Die Lösung wird mit Wasser für Injektionszwecke verdünnt, welches die nötige Menge an Mannitol erhält, so dass eine isotonische Lösung entsteht, die verabreicht werden kann. Nach dem Sterilfiltrieren der Lösung wird diese in Fläschchen (vials) gefüllt und lyophilisiert.

Zur Rekonstitution vor der Verabreichung wird eine Natriumchloridlösung (z.B. 0.1%) verwendet, um so die Aggregation des Peptides und damit auch die Löslichkeit zu kontrollieren. Die Rekonstitution erfolgt gegebenenfalls durch behutsames Schwenken oder Schütteln, wobei eine Schaumbildung zu vermeiden ist.

Die erfindungsgemässen pharmazeutischen Darreichungsformen erlauben die anhaltende Zuführung der Peptidverbindung nach Verabreichung der Darreichungsform bei dem Subjekt. Dauer und Ausmass der Zuführung können durch Änderung der Konzentrationen von Peptidverbindung und die Konzentration des verwendeten Salzes variiert werden.

Desweiteren ist die Standzeit nach Rekonstitution für die Freisetzung des peptidischen Wirkstoffes von Bedeutung. Die Standzeit kann zwischen etwa 0 bis etwa 120 min, vorzugsweise zwischen etwa 10 bis etwa 120 Minuten, besonders bevorzugt zwischen etwa 15 bis 60 Minuten liegen. Es wurde gefunden, dass das durch Aggregation erhaltene kolloidale System sich während der Standzeit verändert und dass sich die Viskosität erhöht. Bei einer Standzeit von mehr als etwa 120 min war keine signifikante Veränderung der Viskosität mehr zu beobachten.

Die erfindungsgemäßen pharmazeutischen Darreichungsformen können vorzugsweise subkutan (s.c.) oder intramuskulär (i.m.) verabreicht werden. Im Falle der intramuskulären Verabreichung erfolgt die injektion beispielsweise in den M. gluteus maximus, vorzugsweise in den äußeren oberen Quadranten des M. gluteus maximus. Im Falle der subkutanen Verabreichung erfolgt die Injektion beispielsweise in die Subkutis des Abdomens.

Die vorliegende Erfindung wird in den nachstehenden Beispielen 1 bis 7 näher beschrieben ohne die Erfindung darauf zu beschränken.

### Beispiel 1

200 g reines D-63153 (kalkuliert als freie Base) werden in 3386,7 g 30 %ige wässriger Essigsäure gelöst, so dass eine klare Lösung entsteht. 438,4 g Mannitol wird zugefügt und unter Rühren aufgelöst. Die Lösung wird mit Wasser für Injektionszwecke auf eine Gesamtmenge von 20320 g aufgefüllt.

Nachdem die Lösung steril gefiltert worden ist, wird sie in 10 ml Portionen in Fläschchen (vials) zur Lyophilisation abgefüllt.

Nach dem Verfahren enthält jedes Fläschchen 100 mg D-63153 (freie Base) und 109,6 mg Mannitol.

Das Lyophilisat wird durch Zugabe von 4 ml 0,1%ige Natriumchloridlösung und behutsames Schütteln (Schaumbildung vermeiden) rekonstituiert, um eine Suspension von 25 mg/ml zu erhalten.

### Beispiel 2

Lyophilisate, die 75 mg D-63153 enthalten, wurden hergestellt und mit 3 ml Lösungsmittel rekonstituiert (25 mg D-63153/ml). Die Rekonstitution erfolgte mit sterilem Wasser für Injektionszwecke (Nicht-Depot Darreichungsform; siehe Tabelle 1) beziehungsweise mit 0,1 % NaCl (Depot Darreichungsform; siehe Tabelle 2). Eine einmalige Dosis von 1,68 mg/kg wurde Beagle Hunden subkutan injiziert. Die D-63153-Plasmaspiegel wurden zu verschiedenen Zeitpunkten nach Verabreichung gemessen.

Durch die Verwendung der Depot-Darreichungsform konnten die Maximum-Plasmaspiegel (Cmax) gesenkt werden, während die Fläche unter der Kurve weitgehend stabil erhalten blieb, welches einen Depoteffekt ergiebt. Die absolute Bioverfügbarkeit blieb weitgehend unverändert und wurde mit 62 % für die Nicht-Depot Darreichungsform, beziehungsweise 64.3% für die Depot-Form berechnet [Schwahn and Romeis, 1999].

### Beispiel 3

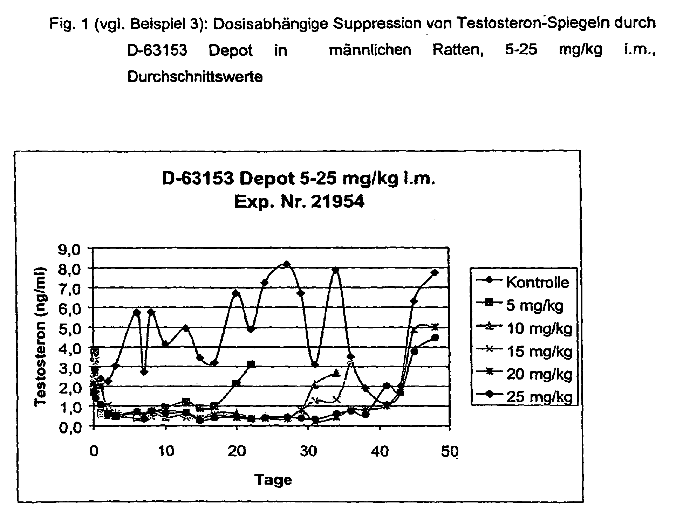

Um das D-63153-Depot auf seine testosteronunterdrückendes Potential zu subrimieren, wurde es männlichen Ratten in 5 verschiedenen Dosen (5-25 mg/kg) intramuskulär (i.m.) injiziert. Die Depot Darreichungsform wurde durch Resuspendieren von D-63153-Lyophilisat in 0,1 %iger steriler NaCl generiert. Der Testosteronspiegel wurde vor der Verabreichung des Medikaments gemessen und jeweils 4 Stunden, 8 Stunden und 24 Stunden danach. Desweitere wurde der Testosteronspiegel in der ersten Woche nach injektion täglich einmal und anschliessend an jedem 2. Tag bestimmt, jeweils so lange, bis der Testosteronwert wieder im normalen Bereich lag. Die Kontrollgruppe wurde nur mit einer Vehikellösung behandelt (siehe Fig. 1).

Eine dosisabhängige Suppression der Testosteronspiegel konnte in allen Gruppen nachgewiesen werden. Die Suppression dauerte von 17 Tagen (5 mg/kg) bis zu 43 Tagen (20 mg/kg) an. Anschliessend lagen die Testosteronwerte innerhalb weniger Tage wieder im Normalbereich.

### Beispiel 4

10 mg-Lyophilisate von D-63153 wurden in 4 ml sterilem Wasser für Injektionszwecke rekonstituiert (Nicht-Depot Darreichungsform, 2,5 mg/ml D-63153, klinische Phase 1a) und 100 mg-Lyophilisate von D-63153 wurden in 4 ml 0,1 % NaCl gelöst (Depot Darreichungsform, 25 mg/ml D-63153, klinische Phase 1 b). Männlichen freiwilligen Versuchspersonen wurden 10 mg pro Person intramuskulär injiziert. An bestimmten Zeitpunkten nach der Verabreichung wurden die Plasmaspiegel von D-63153 gemessen (siehe Tabelle 3).

Die Ergebnisse zeigen das der Depoteffekt sowohl durch niedrigere Cₘᵢₙ und AUC₀-₂₄ Plasmaspiegel als auch durch eine Verlängerung von t_{max,} t_{1/2} und vor allem ein Anstieg der MRT (mean residence time) bestätigt werden kann. Die Depot Darreichungsform hat fast die gleiche AUC₀₋ₜₗₐₛₜ wie die Nicht-Depot Darreichungsform (887,44 ng*h/ml verglichen mit 1165,93 ng*h/ml) wodurch gezeigt wird, dass beide Zusammensetzungen ähnliche biologische Verfügbarkeiten haben. Aus der Depot Darreichungsform wird langsamer freigesetzt, angezeigt durch einen niedrigeren cₘₐₓ Spiegel und einen mehr als doppelt so hohen MRT Wert.

### Beispiel 5

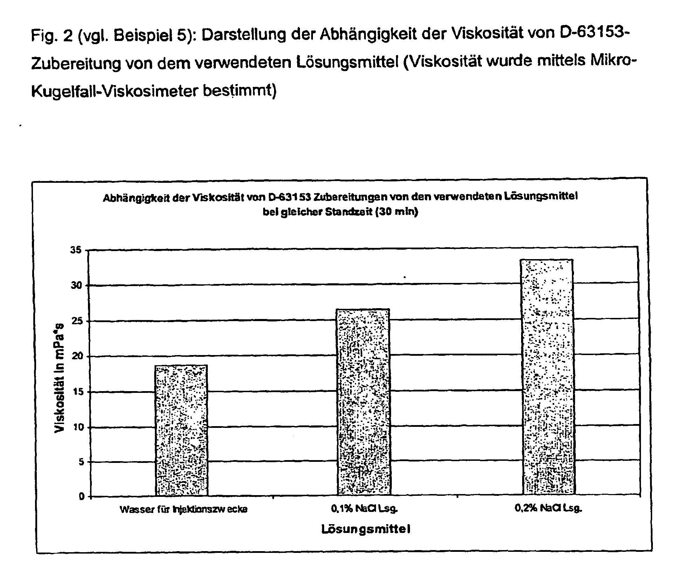

Lyophilisate, die 65 mg und 100 mg D-63153 enthalten, wurden hergestellt und mit Lösungsmittel so rekonstituiert, dass eine Lösung erhalten wird, die eine Konzentration von 25 mg D-63153/ml aufweist. Als Lösungsmittel dienten Wasser für injektionszwecke, 0,1% NaCl-Lösung und 0,2 % NaCl-Lösung. Es wurde das Ausmass der Veränderungen der kolloidalen Eigenschaften der Lösungen anhand von deren Viskositäten untersucht. Die Ergebnisse sind in Fig. 2 zusammengefasst.

### Beispiel 6

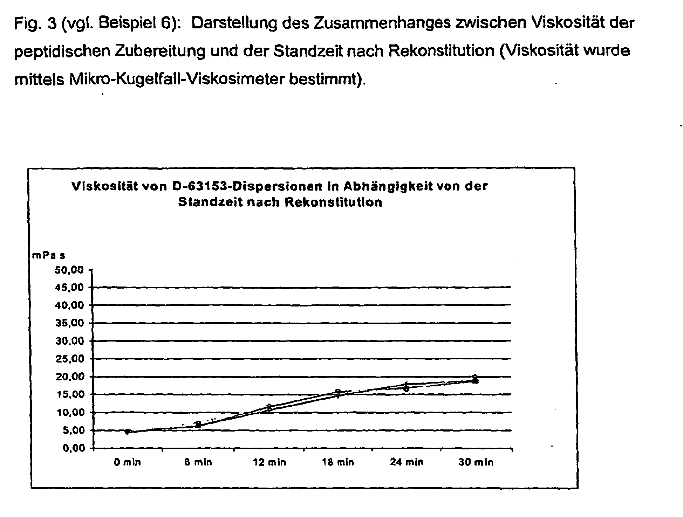

Lyophilisate, die 100 mg D-63153 enthalten, wurden hergestellt und mit Lösungsmittel so rekonstituiert, dass eine Lösung erhalten wird, die eine Konzentration von 25 mg/ml aufweist. Zur Beschreibung der Veränderung des kolloiddispersen Systems, dass nach Rekonstitution entsteht, ist in Fig. 3 die Viskosität in Abhängigkeit von der Standzeit bzw. Lagerzeit nach Rekonstitution dargestellt.

### Beispiel 7

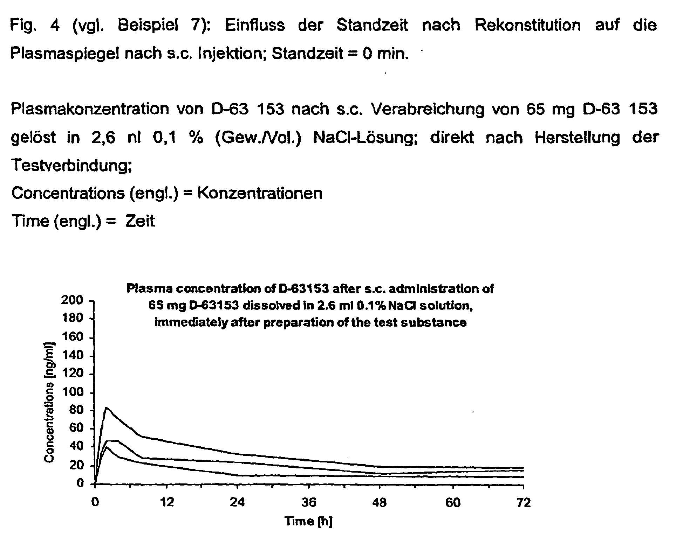

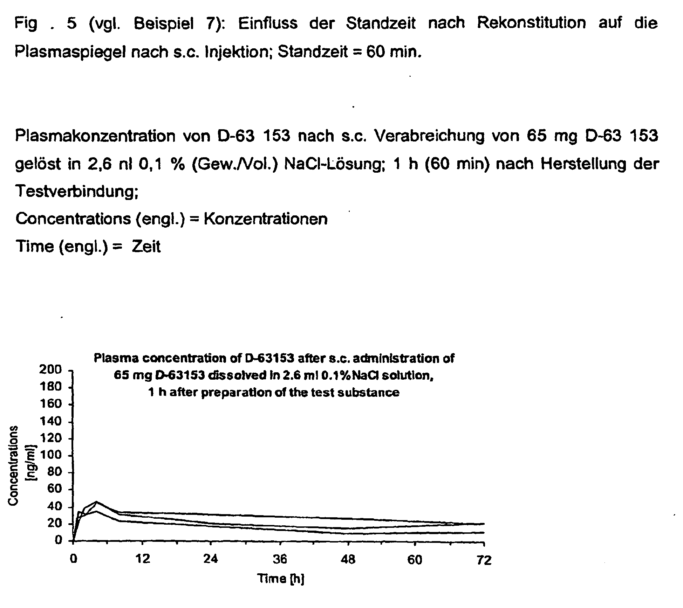

Lyophilisate, die 65 mg D-63153 enthalten, wurden hergestellt und mit 2,6 ml eine 0,1%igen NaCl-Lösung rekonstituiert, die erhaltene Lösung wurde zum einen sofort (Standzeit: 0 Minuten) s.c. an Hunden appliziert (siehe Fig. 4) und zum anderen nach einer Stunde nach Rekonstitution (Standzeit: 60 min) s.c. (siehe Fig. 5) an Hunden appliziert. Die Plasmaspiegel von D-63153 wurden über eine Zeit von 72 Stunden ermittelt.

Das durch Aggregation erhaltene kolloidale System verändert sich während der Standzeit, in dem sich dessen Viskosität erhöht. Damit verbunden ist eine leichte Veränderung der Plasmaspiegelkurven, mit dem Ergebnis, dass die maximale Plasmakonzentration erniedrigt und die Reproduzierbarkeit der Plasmaspiegelkurven verbessert ist.

**Tabelle 1 (vgl. Beispiel 2): Pharmakokinetische Parameter von D-63153 Nicht-Depot Darreichungsform in Beagle Hunden, 1,68 mg/kg s.c.**

| Pharmakokinetische Parameter von D-63153 | | | | | |
|---|---|---|---|---|---|
| | | D-63153 in 5.2% wässrig.Mannitol | | | |
| D = 1.68 mg Peptidbase / kg | | Cₘₐₓ | tₘₐₓ | | AUCₙₒᵣₘ |
| n = 4 | | [ng/ml] | [h] | | [ng·h/ml] |
| | Mittelwert | 216,55 | 5,0 | | 19434,3 |
| | Min | 139,16 | 2,0 | | 15458,0 |
| | Max | 251,90 | 6,0 | | 22103,8 |

**Tabelle 2 (vgl. Beispiel 2): Pharmakokinetische Parameter von D-63153 Depot Darreichungsform in Beagle Hunden, 1,68 mg/kg s.c.**

| Pharmakokinetische Parameter von D-63153 | | | | | |
|---|---|---|---|---|---|
| D = 1.68 mg Peptidbase / kg | | D-63153 in wässr. Mannitol / 0.1% NaCl | | | |
| n = 4 | | Cₘₐₓ | tₘₐₓ | | AUC |
| | | [ng/ml] | [h] | | [ng·h/ml] |
| | Mittelwert | 97,44 | 7,0 | | 17688,2 |
| | Min | 64,75 | 2,0 | | 14445,6 |
| | Max | 199,62 | 8,0 | | 19676,9 |

**Tabelle 3 (vgl. Beispiel 4): Pharmakokinetische Parameter von D-63153: Vergleich zwischen Nicht-Depot und Depot Darreichungsform in männlichen freiwilligen Versuchspersonen, 10 mg/Person (0,14-0,17 mg/kg) i.m.**

| | | | | | | | | |
|---|---|---|---|---|---|---|---|---|
| | | | | | | | | |

| Person | Cₘₐₓ [ng/ml] | tₘₐₓ [h] | tₗₐₛₜ [h] | AUC₀₋ₜₗₐₛₜ [ng*h/ml] | AUC₀₋₂₄ [ng*h/ml] | AUC₀₋₂₄ [%] | t_{1/2} [h] | MRT [h] |
|---|---|---|---|---|---|---|---|---|
| n | 6 | 6 | 6 | 6 | 6 | 6 | 6 | 6 |
| | | | | | | | | |
| Nicht-Depot | 99,90 | 0,50 | 300,00 | 1165,93 | 495,41 | 42,40 | 27,60 | 52,24 |
| | | | | | | | | |
| Depot | 11,02 | 2,50 | 360,00 | 887,44 | 151,05 | 16,7 | 50,05 | 129,36 |

## Patentansprüche

1. Pharmazeutische Gel-Zubereitung, die eine Mischung aus wenigstens einer pharmazeutisch aktiven, ionischen Peptidverbindung mit einer Länge von 8 bis 12 Aminosäuren in lyophilisierter Form bei einer Konzentration von 5 bis 50 mg Peptid pro ml der Gesamtmenge der pharmazeutischen Zubereitung, und einer wässrigen Lösung eines anorganischen oder Essigsäuresalzes in einer Konzentration von 0,01 % bis 0,9 % (Gewicht/Volumen) umfasst, wobei nach dem Vermischen die pharmazeutische Gel-Zubereitung sofort oder nach einer Standzeit von bis zu etwa 120 Minuten, vorzugsweise zwischen etwa 10 min bis etwa 120 min verabreicht werden kann.

2. Pharmazeutische Gel-Zubereitung nach Anspruch 1, **dadurch gekennzeichnet, dass** die pharmazeutisch aktive, ionische Peptidverbindung kationisch ist.

3. Pharmazeutische Gel-Zubereitung nach Anspruch 1, **dadurch gekennzeichnet, dass** die pharmazeutisch aktive, ionische Peptidverbindung anionisch ist.

4. Pharmazeutische Gel-Zubereitung nach Anspruch 1, **dadurch gekennzeichnet, dass** die pharmazeutisch aktive, ionische Peptidverbindung ein mono-, di- oder multivalentes kationisches oder anionisches Peptid ist.

5. Pharmazeutische Gel-Zubereitung nach Anspruch 1, **dadurch gekennzeichnet, dass** die pharmazeutisch aktive, ionische Peptidverbindung ein mono-, di- oder multivalentes ampholytisches Peptid ist.

6. Pharmazeutische Gel-Zubereitung nach einem der Ansprüche 1 bis 5, **dadurch gekennzeichnet, dass** die pharmazeutisch aktive, ionische Peptidverbindung ein GnRH-Analogon ist.

7. Pharmazeutische Gel-Zubereitung nach einem der Ansprüche 1 bis 5, **dadurch gekennzeichnet, dass** die pharmazeutisch aktive, ionische Peptidverbindung ein GnRH-Antagonist ist.

8. Pharmazeutische Gel-Zubereitung nach einem der Ansprüche 1 bis 7, **dadurch gekennzeichnet, dass** die pharmazeutisch aktive, ionische Peptidverbindung Cetrorelix, Teverelix, Abarelix, Ganirelix, Azaline B, Antide, Detirelix, Ramorelix, Degarefix, D-63153, ein pharmazeutisch aktives Salz einer dieser Verbindungen oder ein Gemisch von diesen ist.

9. Pharmazeutische Gel-Zubereitung nach einem der Ansprüche 1 bis 8, **dadurch gekennzeichnet, dass** die pharmazeutisch aktive, ionische Peptidverbindung der GnRH-Antagonist D-63153 ist.

10. Pharmazeutische Gel-Zubereitung nach einem der vorhergehenden Ansprüche, **dadurch gekennzeichnet, dass** es sich bei dem anorganischen oder dem Essigsäuresalz um ein physiologisch verträgliches Salz handelt.

11. Pharmazeutische Gel-Zubereitung nach einem der vorhergehenden Ansprüche, **dadurch gekennzeichnet, dass** das wässrige anorganische oder Essigsäuresalz aus der Gruppe ausgewählt worden ist, die Natriumchlorid, Kalziumchlorid, Magnesiumchlorid, Natriumacetat, Kalziumacetat und Magnesiumacetat umfasst.

12. Pharmazeutische Gel-Zubereitung nach einem der vorhergehenden Ansprüche, **dadurch gekennzeichnet, dass** die Mischung aus der pharmazeutisch aktiven, ionischen Peptidverbindung und der wässrigen Lösung des anorganischen oder des Essigsäuresalzes eine flüssige Suspension oder eine halbfeste Dispersion ist.

13. Pharmazeutische Gel-Zubereitung nach einem der vorhergehenden Ansprüche, **dadurch gekennzeichnet, dass** die Konzentration der pharmazeutisch aktiven, ionischen Peptidverbindung im Bereich von etwa 10 mg bis etwa 50 mg pro ml der Gesamtmenge der pharmazeutischen Gel-Zubereitung liegt.

14. Pharmazeutische Gel-Zubereitung nach einem der vorhergehenden Ansprüche, **dadurch gekennzeichnet, dass** die Konzentration der pharmazeutisch aktiven, ionischen Peptidverbindung im Bereich von etwa 20 mg bis etwa 30 mg pro ml der Gesamtmenge der pharmazeutischen Gel-Zubereitung liegt.

15. Pharmazeutische Gel-Zubereitung nach einem der vorhergehenden Ansprüche, **dadurch gekennzeichnet, dass** die Konzentration der pharmazeutisch aktiven, ionischen Peptidverbindung im Bereich von etwa 25 mg pro ml der Gesamtmenge der pharmazeutischen Gel-Zubereitung liegt.

16. Pharmazeutische Gel-Zubereitung nach einem der vorhergehenden Ansprüche, **dadurch gekennzeichnet, dass** D-63153 die pharmazeutisch aktive, ionische Peptidverbindung ist und dass die Konzentration im Bereich von etwa 5 mg bis etwa 50 mg pro ml der Gesamtmenge der pharmazeutischen Gel-Zu-bereitung liegt.

17. Pharmazeutische Gel-Zubereitung nach einem der vorhergehenden Ansprüche, **dadurch gekennzeichnet, dass** D-63153 die pharmazeutisch aktive, ionische Peptidverbindung ist und dass die Konzentration im Bereich von etwa 10 mg bis etwa 50 mg pro ml der Gesamtmenge der pharmazeutischen Gel-Zubereitung liegt.

18. Pharmazeutische Gel-Zubereitung nach einem der vorhergehenden Ansprüche, **dadurch gekennzeichnet, dass** D-63153 die pharmazeutisch aktive, ionische Peptidverbindung ist und dass die Konzentration im Bereich von etwa 20 mg bis etwa 30 mg pro ml der Gesamtmenge der pharmazeutischen Gel-Zubereitung liegt.

19. Pharmazeutische Gel-Zubereitung nach einem der vorhergehenden Ansprüche, **dadurch gekennzeichnet, dass** D-63153 die pharmazeutisch aktive, ionische Peptidverbindung ist und das die Konzentration im Bereich von etwa 25 mg pro ml der Gesamtmenge der pharmazeutischen Gel-Zubereitung liegt.

20. Pharmazeutische Gel-Zubereitung nach einem der vorhergehenden Ansprüche, **dadurch gekennzeichnet, dass** die Konzentration des anorganischen oder des Essigsäuresalzes im Bereich von etwa 0,05 % bis etwa 0,5 % (Gewicht/Volumen) liegt.

21. Pharmazeutische Gel-Zubereitung nach einem der vorhergehenden Ansprüche, **dadurch gekennzeichnet, dass** die Konzentration der anorganischen oder Essigsäuresalz-Lösung etwa 0,1 % (Gewicht/Volumen) beträgt.

22. Pharmazeutische Gel-Zubereitung nach einem der vorhergehenden Ansprüche, **dadurch gekennzeichnet, dass** das anorganische Salz Natriumchlorid ist und dass die Konzentration im Bereich von etwa 0,01 % bis etwa 0,9 % (GewichWolumen) liegt.

23. Pharmazeutische Gel-Zubereitung nach einem der vorhergehenden Ansprüche, **dadurch gekennzeichnet, dass** das anorganische Salz Natriumchlorid ist und dass die Konzentration im Bereich von etwa 0,05 % bis etwa 0,5 % (Gewicht/Volumen) liegt.

24. Pharmazeutische Gel-Zubereitung nach einem der vorhergehenden Ansprüche, **dadurch gekennzeichnet, dass** das anorganische Salz Natriumchlorid ist und das die Konzentration etwa 0,1 % (Gewicht/Volumen) beträgt.

25. Pharmazeutische Gel-Zubereitung nach einem der vorhergehenden Ansprüche, **dadurch gekennzeichnet, dass** zumindest eine der pharmazeutisch aktiven, ionischen Peptidverbindungen D-63153 ist und dass das anorganische Salz Natriumchlorid ist.

26. Pharmazeutische Gel-Zubereitung nach einem der vorhergehenden Ansprüche, **dadurch gekennzeichnet, dass** zumindest eine der pharmazeutisch aktiven, ionischen Peptidverbindungen D-63153 ist, dass deren Konzentration etwa 25 ml pro ml der Zubereitung beträgt und dass das anorganische Salz Natriumchlorid ist, dessen Konzentration etwa 0,1 % (Gewicht/Volumen) beträgt.

27. Pharmazeutische Gel-Zubereitung nach einem der vorhergehenden Ansprüche, **dadurch gekennzeichnet, dass** die Mischung aus der pharmazeutisch aktiven, ionischen Peptidverbindung und der wässrigen Lösung des anorganischen oder des Essigsäuresalzes eine molekulare Dispersion oder eine kolloidale Mischung ist, die eine flüssige oder halbfeste Konsistent besitzen kann.

28. Pharmazeutische Gel-Zubereitung nach einem der vorhergehenden Ansprüche, **dadurch gekennzeichnet, dass** eine kolloidale Dispersion durch Rekonstitution gebildet ist.

29. Pharmazeutische Gel-Zubereitung nach einem der vorhergehenden Ansprüche, **dadurch gekennzeichnet, dass** eine kolloidale Dispersion durch Lagerung oder Stehen lassen nach der Rekonstitution gebildet ist und ihre Viskosität in Abhängigkeit von der Zeit ändert, und somit die Reproduzierbarkeit der verzögerten Freisetzung des aktiven Bestandteils verbessert.

30. Verfahren zur Herstellung einer pharmazeutischen Gel-Zubereitung, das den Schritt umfasst,
dass eine Menge von wenigstens einer pharmazeutisch aktiven Peptidverbindung, die eine Länge von 8 bis 12 Aminosäuren aufweist und in lyophilisierter Form vorliegt und eine wässrige Lösung eines anorganischen oder Essigsäuresalzes zusammengemischt werden, **dadurch gekennzeichnet, dass** die wenigstens eine pharmazeutisch aktive Peptidverbindung in einer Konzentration von 5 mg bis 50 mg Peptid pro ml der Gesamtmenge der pharmazeutischen Zubereitung vorliegt und dass die wässrige Lösung eines anorganischen oder Essigsäuresalzes in einer Konzentration von 0,01 % bis 0,9 % (Gewicht/Volumen) vorliegt.

31. Verfahren zur Herstellung einer pharmazeutischen Gel-Zubereitung nach Anspruch 30, **dadurch gekennzeichnet, dass** die pharmazeutisch aktive, ionische Peptidverbindung D-63153 ist und dass das anorganische Salz Natriumchlorid ist.

32. Verfahren zur Herstellung einer pharmazeutischen Gel-Zubereitung nach Anspruch 30, **dadurch gekennzeichnet, dass** die pharmazeutisch aktive, ionische Peptidverbindung D-63153 ist, deren Menge etwa 25 mg/ml beträgt, und dass das anorganische Salz Natriumchlorid ist, dessen Konzentration etwa 0,1 % (Gewicht/Volumen) beträgt.

33. Verfahren zur Herstellung einer pharmazeutischen Gel-Zubereitung nach einem der Ansprüche 30 bis 32, das weiterhin den Schritt der Sterilisierung der pharmazeutischen Gel-Zubereitung durch Bestrahlung mit Gammastrahlen oder Elektronenstrahlen umfasst.

34. Verfahren zur Herstellung einer pharmazeutischen Gel-Zubereitung nach einem der vorhergehenden Ansprüche, bei dem die Herstellung der Peptidformulierung unter Anwendung aseptischer Verfahrensweisen erfolgt.

35. Kit zur Herstellung einer pharmazeutischen Gel-Zubereitung, umfassend eine vorher festgelegte Menge einer pharmazeutisch aktiven, ionischen Peptidverbindung in lyophilisierter Form und einer wässrigen Lösung eines anorganischen oder Essigsäuresalzes, wobei die wenigstens eine pharmazeutisch aktive Peptidverbindung eine Länge von 8 bis 12 Aminosäuren aufweist und die wässrige Lösung eines anorganischen oder Essigsäuresalzes in einer Konzentration von 0,01 % bis 0,9 % (Gewicht/Volumen) vorliegt, **dadurch gekennzeichnet, dass** bei der Rekonstitution mit der wässrigen Lösung die wenigstens eine pharmazeutisch aktive Peptidverbindung in einer Konzentration von 5 mg bis 50 mg Peptid pro ml der Gesamtmenge der pharmazeutischen Zubereitung vorliegt.

36. Kit nach Anspruch 35, **dadurch gekennzeichnet, dass** die pharmazeutisch aktive Peptidverbindung D-63153 in lyophilisierter Form vorliegt.

37. Kit nach Anspruch 35, **dadurch gekennzeichnet, dass** das D-63153 Lyophilisat zusätzlich Mannit enthält.

38. Kit nach Anspruch 35, **dadurch gekennzeichnet, dass** das anorganische Salz Natriumchlorid ist.

39. Kit nach einem der Ansprüche 35 bis 38, **dadurch gekennzeichnet, dass** die Menge von D-63153 etwa 25 mg pro fertiger Zubereitung und die Konzentration der wässrigen Natriumchloridlösung etwa 0,1 % (Gewicht/Volumen) beträgt.

40. Pharmazeutische Gel-Zubereitung nach einem der Ansprüche 1 bis 29 zur Verwendung bei der Behandlung einer hormonabhängigen Störung bei einem Patienten.

41. Pharmazeutische Gel-Zubereitung nach Anspruch 40, **dadurch gekennzeichnet, dass** die hormonabhängige Störung Prostatakrebs, Brustkrebs, en Uterusmyom, Endometriose oder Pubertas Precox ist.

42. Pharmazeutische Gel-Zubereitung nach einem der Ansprüche 1 bis 29 zur Verwendung bei der Modifizierung der Reproduktionsfunktion eines Patienten.

43. Pharmazeutische Gel-Zubereitung nach einem der Ansprüche 40 bis 42 **dadurch gekennzeichnet, dass** die pharmazeutische Gel-Zubereitung dem Patienten subkutan oder intramuskulär verabreicht werden kann.

44. Pharmazeutische Gel-Zubereitung nach einem der Ansprüche 40 bis 42, **dadurch gekennzeichnet, dass** die verabreichte pharmazeutische Gel-Zubereitung eine anhaltende pharmazeutische Aktivität aufweist.

45. Pharmazeutische Gel-Zubereitung nach Anspruch 44, **dadurch gekennzeichnet, dass** die verabreichte pharmazeutische Gel-Zubereitung über mindestens 4 Wochen hinweg eine anhaltende pharmazeutische Aktivität aufweist.

46. Pharmazeutische Gel-Zubereitung nach Anspruch 44, **dadurch gekennzeichnet, dass** die verabreichte pharmazeutische Gel-Zubereitung über mindestens 8 Wochen hinweg eine anhaltende pharmazeutische Aktivität aufweist.

47. Pharmazeutische Gel-Zubereitung nach Anspruch 44, **dadurch gekennzeichnet, dass** die verabreichte pharmazeutische Gel-Zubereitung über mindestens 12 Wochen hinweg eine anhaltende pharmazeutische Aktivität aufweist.

## Claims

1. Pharmaceutical gel preparation which comprises a mixture of at least one pharmaceutically active, ionic peptide compound having a length of from 8 to 12 amino acids in lyophilised form at a concentration of from 5 to 50 mg of peptide per ml of the total amount of the pharmaceutical preparation and an aqueous solution of an inorganic or acetic acid salt in a concentration of from 0.01 % to 0.9 % (weight/volume), wherein after mixing the pharmaceutical gel preparation can be administered immediately or after a standing time of up to about 120 minutes, preferably between about 10 min to about 120 min.

2. Pharmaceutical gel preparation according to claim 1, **characterised in that** the pharmaceutically active, ionic peptide compound is cationic.

3. Pharmaceutical gel preparation according to claim 1, **characterised in that** the pharmaceutically active, ionic peptide compound is anionic.

4. Pharmaceutical gel preparation according to claim 1, **characterised in that** the pharmaceutically active, ionic peptide compound is a mono-, di- or multivalent cationic or anionic peptide.

5. Pharmaceutical gel preparation according to claim 1, **characterised in that** the pharmaceutically active, ionic peptide compound is a mono-, di- or multivalent ampholytic peptide.

6. Pharmaceutical gel preparation according to any of claims 1 to 5, **characterised in that** the pharmaceutically active, ionic peptide compound is a GnRH analogue.

7. Pharmaceutical gel preparation according to any of claims 1 to 5, **characterised in that** the pharmaceutically active, ionic peptide compound is a GnRH antagonist.

8. Pharmaceutical gel preparation according to any of claims 1 to 7, **characterised in that** the pharmaceutically active, ionic peptide compound is cetrorelix, teverelix, abarelix, ganirelix, azaline B, antide, detirelix, ramorelix, degarelix, D-63153, a pharmaceutically active salt of one of these compounds or a mixture of these.

9. Pharmaceutical gel preparation according to any of claims 1 to 8, **characterised in that** the pharmaceutically active, ionic peptide compound is the GnRH antagonist D-63153.

10. Pharmaceutical gel preparation according to any of the preceding claims, **characterised in that** the inorganic or the acetic acid salt is a physiologically acceptable salt.

11. Pharmaceutical gel preparation according to any of the preceding claims, **characterised in that** the aqueous inorganic or acetic acid salt has been chosen from the group which comprises sodium chloride, calcium chloride, magnesium chloride, sodium acetate, calcium acetate and magnesium acetate.

12. Pharmaceutical gel preparation according to any of the preceding claims, **characterised in that** the mixture of the pharmaceutically active, ionic peptide compound and the aqueous solution of the inorganic or the acetic acid salt is a liquid suspension or a semi-solid dispersion.

13. Pharmaceutical gel preparation according to any of the preceding claims, **characterised in that** the concentration of the pharmaceutically active, ionic peptide compound is in the range of from about 10 mg to about 50 mg per ml of the total amount of the pharmaceutical gel preparation.

14. Pharmaceutical gel preparation according to any of the preceding claims, **characterised in that** the concentration of the pharmaceutically active, ionic peptide compound is in the range of from about 20 mg to about 30 mg per ml of the total amount of the pharmaceutical gel preparation.

15. Pharmaceutical gel preparation according to any of the preceding claims, **characterised in that** the concentration of the pharmaceutically active, ionic peptide compound is in the region of about 25 mg per ml of the total amount of the pharmaceutical gel preparation.

16. Pharmaceutical gel preparation according to any of the preceding claims, **characterised in that** D-63153 is the pharmaceutically active, ionic peptide compound and **in that** the concentration is in the range of from about 5 mg to about 50 mg per ml of the total amount of the pharmaceutical gel preparation.

17. Pharmaceutical gel preparation according to any of the preceding claims, **characterised in that** D-63153 is the pharmaceutically active, ionic peptide compound and **in that** the concentration is in the range of from about 10 mg to about 50 mg per ml of the total amount of the pharmaceutical gel preparation.

18. Pharmaceutical gel preparation according to any of the preceding claims, **characterised in that** D-63153 is the pharmaceutically active, ionic peptide compound and **in that** the concentration is in the range of from about 20 mg to about 30 mg per ml of the total amount of the pharmaceutical gel preparation.

19. Pharmaceutical gel preparation according to any of the preceding claims, **characterised in that** D-63153 is the pharmaceutically active, ionic peptide compound and **in that** the concentration is in the region of about 25 mg per ml of the total amount of the pharmaceutical gel preparation.

20. Pharmaceutical gel preparation according to any of the preceding claims, **characterised in that** the concentration of the inorganic or the acetic acid salt is in the range of from about 0.05 % to about 0.5 % (weight/volume).

21. Pharmaceutical gel preparation according to any of the preceding claims, **characterised in that** the concentration of the inorganic or acetic acid salt solution is about 0.1 % (weight/volume).

22. Pharmaceutical gel preparation according to any of the preceding claims, **characterised in that** the inorganic salt is sodium chloride and **in that** the concentration is in the range of from about 0.01 % to about 0.9 % (weight/volume).

23. Pharmaceutical gel preparation according to any of the preceding claims, **characterised in that** the inorganic salt is sodium chloride and **in that** the concentration is in the range of from about 0.05 % to about 0.5 % (weight/volume).

24. Pharmaceutical gel preparation according to any of the preceding claims, **characterised in that** the inorganic salt is sodium chloride and **in that** the concentration is about 0.1 % (weight/volume).

25. Pharmaceutical gel preparation according to any of the preceding claims, **characterised in that** at least one of the pharmaceutically active, ionic peptide compounds is D-63153 and **in that** the inorganic salt is sodium chloride.

26. Pharmaceutical gel preparation according to any of the preceding claims, **characterised in that** at least one of the pharmaceutically active, ionic peptide compounds is D-63153, **in that** the concentration thereof is about 25 ml per ml of the preparation and **in that** the inorganic salt is sodium chloride, the concentration of which is about 0.1 % (weight/volume).

27. Pharmaceutical gel preparation according to any of the preceding claims, **characterised in that** the mixture of the pharmaceutically active, ionic peptide compound and the aqueous solution of the inorganic or the acetic acid salt is a molecular dispersion or a colloidal mixture which can have a liquid or semi-solid consistency.

28. Pharmaceutical gel preparation according to any of the preceding claims, **characterised in that** a colloidal dispersion is formed by reconstitution.

29. Pharmaceutical gel preparation according to any of the preceding claims, **characterised in that** a colloidal dispersion is formed by storage or leaving to stand after the reconstitution and its viscosity changes as a function of time, and the reproducibility of the delayed release of the active constituent is thus improved.

30. Process for the production of a pharmaceutical gel preparation which includes the step that an amount of at least one pharmaceutically active peptide compound which has a length of from 8 to 12 amino acids and is present in lyophilised form and an aqueous solution of an inorganic or acetic acid salt are mixed together, **characterised in that** the at least one pharmaceutically active peptide compound is present in a concentration of from 5 mg to 50 mg of peptide per ml of the total amount of the pharmaceutical preparation and **in that** the aqueous solution of an inorganic or acetic acid salt is present in a concentration of from 0.01 % to 0.9 % (weight/volume).

31. Process for the production of a pharmaceutical gel preparation according to claim 30, **characterised in that** the pharmaceutically active, ionic peptide compound is D-63153 and **in that** the inorganic salt is sodium chloride.

32. Process for the production of a pharmaceutical gel preparation according to claim 30, **characterised in that** the pharmaceutically active, ionic peptide compound is D-63153, the amount of which is about 25 mg/ml, and **in that** the inorganic salt is sodium chloride, the concentration of which is about 0.1 % (weight/volume).

33. Process for the production of a pharmaceutical gel preparation according to any of claims 30 to 32, which furthermore includes the step of sterilisation of the pharmaceutical gel preparation by irradiation with gamma rays or electron beams.

34. Process for the production of a pharmaceutical gel preparation according to any of the preceding claims, in which the preparation of the peptide formulation is carried out using aseptic procedures.

35. Kit for the production of a pharmaceutical gel preparation, comprising a predetermined amount of a pharmaceutically active, ionic peptide compound in lyophilised form and of an aqueous solution of an inorganic or acetic acid salt, wherein the at least one pharmaceutically active peptide compound has a length of from 8 to 12 amino acids and the aqueous solution of an inorganic or acetic acid salt is present in a concentration of from 0.01 % to 0.9 % (weight/volume), **characterised in that** in the reconstitution with the aqueous solution the at least one pharmaceutically active peptide compound is present in a concentration of from 5 mg to 50 mg of peptide per ml of the total amount of the pharmaceutical preparation.

36. Kit according to claim 35, **characterised in that** the pharmaceutically active peptide compound D-63153 is present in lyophilised form.

37. Kit according to claim 35, **characterised in that** the D-63153 lyophilisate additionally comprises mannitol.

38. Kit according to claim 35, **characterised in that** the inorganic salt is sodium chloride.

39. Kit according to any of claims 35 to 38, **characterised in that** the amount of D-63153 is about 25 mg per finished preparation and the concentration of the aqueous sodium chloride solution is about 0.1 % (weight/volume).

40. Pharmaceutical gel preparation according to any of claims 1 to 29 for use in the treatment of a hormone-related disorder in a patient.

41. Pharmaceutical gel preparation according to claim 40, **characterised in that** the hormone-related disorder is prostate cancer, breast cancer, a uterine myoma, endometriosis or pubertas precox.

42. Pharmaceutical gel preparation according to any of claims 1 to 29 for use in the modification of the reproduction function of a patient.

43. Pharmaceutical gel preparation according to any of claims 40 to 42, **characterised in that** the pharmaceutical gel preparation can be administered to the patient subcutaneously or intramuscularly.

44. Pharmaceutical gel preparation according to any of claims 40 to 42, **characterised in that** the pharmaceutical gel preparation administered has a sustained pharmaceutical activity.

45. Pharmaceutical gel preparation according to claim 44, **characterised in that** the pharmaceutical gel preparation administered has a sustained pharmaceutical activity over at least 4 weeks.

46. Pharmaceutical gel preparation according to claim 44, **characterised in that** the pharmaceutical gel preparation administered has a sustained pharmaceutical activity over at least 8 weeks.

47. Pharmaceutical gel preparation according to claim 44, **characterised in that** the pharmaceutical gel preparation administered has a sustained pharmaceutical activity over at least 12 weeks.

## Revendications

1. Préparation pharmaceutique sous forme de gel, qui comprend un mélange d'au moins un composé peptidique ionique pharmaceutiquement actif d'une longueur de 8 à 12 acides aminés sous forme lyophilisée à une concentration de 5 à 50 mg de peptide par ml de la quantité totale de la préparation pharmaceutique, et d'une solution aqueuse d'un sel inorganique ou d'un sel d'acide acétique à une concentration de 0,01 % à 0,9 % (poids/volume), la préparation pharmaceutique sous forme de gel pouvant, après le mélange, être administrée immédiatement ou après un temps de repos allant jusqu'à environ 120 minutes, de préférence entre environ 10 minutes et environ 120 minutes.

2. Préparation pharmaceutique sous forme de gel selon la revendication 1, **caractérisée en ce que** le composé peptidique ionique pharmaceutiquement actif est cationique.

3. Préparation pharmaceutique sous forme de gel selon la revendication 1, **caractérisée en ce que** le composé peptidique ionique pharmaceutiquement actif est anionique.

4. Préparation pharmaceutique sous forme de gel selon la revendication 1, **caractérisée en ce que** le composé peptidique ionique pharmaceutiquement actif est un peptide cationique ou anionique monovalent, divalent ou multivalent.

5. Préparation pharmaceutique sous forme de gel selon la revendication 1, **caractérisée en ce que** le composé peptidique ionique pharmaceutiquement actif est un peptide ampholytique monovalent, divalent ou multivalent.

6. Préparation pharmaceutique sous forme de gel selon l'une des revendications 1 à 5, **caractérisée en ce que** le composé peptidique ionique pharmaceutiquement actif est un analogue de la GnRH.

7. Préparation pharmaceutique sous forme de gel selon l'une des revendications 1 à 5, **caractérisée en ce que** le composé peptidique ionique pharmaceutiquement actif est un antagoniste de la GnRH.

8. Préparation pharmaceutique sous forme de gel selon l'une des revendications 1 à 7, **caractérisée en ce que** le composé peptidique ionique, pharmaceutiquement actif est le cetrorelix, le teverelix, l'abarelix, le ganirelix, l'azaline B, l'antide, le detirelix, le ramorelix, le degarefix, le D-63153, un sel pharmaceutiquement actif d'un de ces composés ou un mélange de ceux-ci.

9. Préparation pharmaceutique sous forme de gel selon l'une des revendications 1 à 8, **caractérisée en ce que** le composé peptidique ionique pharmaceutiquement actif est l'antagoniste de la GnRH D-63153.

10. Préparation pharmaceutique sous forme de gel selon l'une des revendications précédentes, **caractérisée en ce que**, dans le cas du sel inorganique ou du sel d'acide acétique, il s'agit d'un sel physiologiquement acceptable.

11. Préparation pharmaceutique sous forme de gel selon l'une des revendications précédentes, **caractérisée en ce que** le sel d'acide acétique ou le sel inorganique aqueux est choisi dans le groupe comprenant le chlorure de sodium, le chlorure de calcium, le chlorure de magnésium, l'acétate de sodium, l'acétate de calcium et l'acétate de magnésium.

12. Préparation pharmaceutique sous forme de gel selon l'une des revendications précédentes, **caractérisée en ce que** le mélange du composé peptidique ionique pharmaceutiquement actif et de la solution aqueuse du sel inorganique ou du sel d'acide acétique est une suspension liquide ou une dispersion semi-solide.

13. Préparation pharmaceutique sous forme de gel selon l'une des revendications précédentes, **caractérisée en ce que** la concentration du composé peptidique ionique pharmaceutiquement actif se situe dans la plage d'environ 10 mg à environ 50 mg par ml de la quantité totale de la préparation pharmaceutique sous forme de gel.

14. Préparation pharmaceutique sous forme de gel selon l'une des revendications précédentes, **caractérisée en ce que** la concentration du composé peptidique ionique pharmaceutiquement actif se situe dans la plage d'environ 20 mg à environ 30 mg par ml de la quantité totale de la préparation pharmaceutique sous forme de gel.

15. Préparation pharmaceutique sous forme de gel selon l'une des revendications précédentes, **caractérisée en ce que** la concentration du composé peptidique ionique pharmaceutiquement actif se situe dans la plage d'environ 25 mg par ml de la quantité totale de la préparation pharmaceutique sous forme de gel.

16. Préparation pharmaceutique sous forme de gel selon l'une des revendications précédentes, **caractérisée en ce que** D-63153 est le composé peptidique ionique pharmaceutiquement actif et **en ce que** la concentration se situe dans la plage d'environ 5 mg à environ 50 mg par ml de la quantité totale de la préparation pharmaceutique sous forme de gel.

17. Préparation pharmaceutique sous forme de gel selon l'une des revendications précédentes, **caractérisée en ce que** D-63153 est le composé peptidique ionique pharmaceutiquement actif et **en ce que** la concentration se situe dans la plage d'environ 10 mg à environ 50 mg par ml de la quantité totale de la préparation pharmaceutique sous forme de gel.

18. Préparation pharmaceutique sous forme de gel selon l'une des revendications précédentes, **caractérisée en ce que** D-63153 est le composé peptidique ionique pharmaceutiquement actif et **en ce que** la concentration se situe dans la plage d'environ 20 mg à environ 30 mg par ml de la quantité totale de la préparation pharmaceutique sous forme de gel.

19. Préparation pharmaceutique sous forme de gel selon l'une des revendications précédentes, **caractérisée en ce que** D-63153 est le composé peptidique ionique pharmaceutiquement actif et **en ce que** la concentration se situe dans la plage d'environ 25 mg par ml de la quantité totale de la préparation pharmaceutique sous forme de gel.

20. Préparation pharmaceutique sous forme de gel selon l'une des revendications précédentes, **caractérisée en ce que** la concentration du sel inorganique ou du sel d'acide acétique se situe dans la plage d'environ 0,05 % à environ 0,5 % (poids/volume).

21. Préparation pharmaceutique sous forme de gel selon l'une des revendications précédentes, **caractérisée en ce que** la concentration de la solution de sel inorganique ou de sel d'acide acétique est d'environ 0,1 % (poids/volume).

22. Préparation pharmaceutique sous forme de gel selon l'une des revendications précédentes, **caractérisée en ce que** le sel inorganique est le chlorure de sodium et **en ce que** la concentration se situe dans la plage d'environ 0,01 % à environ 0,9 % (poids/volume).

23. Préparation pharmaceutique sous forme de gel selon l'une des revendications précédentes, **caractérisée en ce que** le sel inorganique est le chlorure de sodium et **en ce que** la concentration se situe dans la plage d'environ 0,05 % à environ 0,5 % (poids/volume).

24. Préparation pharmaceutique sous forme de gel selon l'une des revendications précédentes, **caractérisée en ce que** le sel inorganique est le chlorure de sodium et **en ce que** la concentration est d'environ 0,1 % (poids/volume).

25. Préparation pharmaceutique sous forme de gel selon l'une des revendications précédentes, **caractérisée en ce qu'**au moins un des composés peptidiques ioniques pharmaceutiquement actifs est le D-63153 et **en ce que** le sel inorganique est le chlorure de sodium.

26. Préparation pharmaceutique sous forme de gel selon l'une des revendications précédentes, **caractérisée en ce qu'**au moins un des composés peptidiques ioniques pharmaceutiquement actifs est le D-63153, **en ce que** sa concentration est d'environ 25 ml par ml de préparation et **en ce que** le sel inorganique est le chlorure de sodium dont la concentration est d'environ 0,1 % (poids/volume).

27. Préparation pharmaceutique sous forme de gel selon l'une des revendications précédentes, **caractérisée en ce que** le mélange du composé peptidique ionique pharmaceutiquement actif et de la solution aqueuse du sel inorganique ou du sel d'acide acétique est une dispersion moléculaire ou un mélange colloïdal qui peut posséder une consistance liquide ou semi-solide.

28. Préparation pharmaceutique sous forme de gel selon l'une des revendications précédentes, **caractérisée en ce qu'**une dispersion colloïdale est formée par reconstitution.

29. Préparation pharmaceutique sous forme de gel selon l'une des revendications précédentes, **caractérisée en ce qu'**une dispersion colloïdale est formée par stockage ou par mise au repos après la reconstitution et sa viscosité change en fonction du temps et améliore donc la reproductibilité de la libération retardée du composant actif.

30. Procédé de fabrication d'une préparation pharmaceutique sous forme de gel qui comprend l'étape selon laquelle
une quantité d'au moins un composé peptidique ionique pharmaceutiquement actif qui présente une longueur de 8 à 12 acides aminés et qui se présente sous forme lyophilisée et une solution aqueuse d'un sel inorganique ou d'un sel d'acide acétique sont mélangées l'une avec l'autre, **caractérisé en ce que** le au moins un composé peptidique ionique pharmaceutiquement actif est présent à une concentration de 5 mg à 50 mg de peptide par ml de la quantité totale de la préparation pharmaceutique, et **en ce que** la solution aqueuse d'un sel inorganique ou d'un sel d'acide acétique est présente à une concentration de 0,01 % à 0,9 % (poids/volume).

31. Procédé de fabrication d'une préparation pharmaceutique sous forme de gel selon la revendication 30, **caractérisé en ce que** le composé peptidique ionique pharmaceutiquement actif est le D-63153 et **en ce que** le sel inorganique est le chlorure de sodium.

32. Procédé de fabrication d'une préparation pharmaceutique sous forme de gel selon la revendication 30, **caractérisé en ce que** le composé peptidique ionique pharmaceutiquement actif est le D-63153, dont la quantité est d'environ 25 mg/ml, et **en ce que** le sel inorganique est le chlorure de sodium dont la concentration est d'environ 0,1 % (poids/volume).

33. Procédé de fabrication d'une préparation pharmaceutique sous forme de gel selon l'une des revendications 30 à 32, qui comprend en outre l'étape de stérilisation de la préparation pharmaceutique sous forme de gel par irradiation avec des rayons gamma ou des faisceaux d'électrons.

34. Procédé de fabrication d'une préparation pharmaceutique sous forme de gel selon l'une des revendications précédentes, dans lequel la fabrication de la formulation peptidique s'effectue en utilisant des procédures aseptiques.

35. Kit de fabrication d'une préparation pharmaceutique sous forme de gel, comprenant une quantité préalablement déterminée d'un composé peptidique pharmaceutiquement actif sous forme lyophilisée et d'une solution aqueuse d'un sel inorganique ou d'un sel d'acide acétique, le au moins un composé peptidique pharmaceutiquement actif présentant une longueur de 8 à 12 acides aminés, et la solution aqueuse d'un sel inorganique ou d'un sel d'acide acétique étant présente à une concentration de 0,01 % à 0,9 % (poids/volume), **caractérisé en ce que**, lors de la reconstitution avec la solution aqueuse, le au moins un composé peptidique ionique pharmaceutiquement actif est présent à une concentration de 5 mg à 50 mg de peptide par ml de la quantité totale de la préparation pharmaceutique.

36. Kit selon la revendication 35, **caractérisé en ce que** le composé peptidique pharmaceutiquement actif D-63153 se présente sous forme lyophilisée.

37. Kit selon la revendication 35, **caractérisé en ce que** le lyophilisat D-63153 contient en plus du mannitol.

38. Kit selon la revendication 35, **caractérisé en ce que** le sel inorganique est le chlorure de sodium.

39. Kit selon l'une des revendications 35 à 38, **caractérisé en ce que** la quantité de D-63153 est d'environ 25 mg par préparation terminée et **en ce que** la concentration de la solution aqueuse de chlorure de sodium est d'environ 0,1 % (poids/volume).

40. Préparation pharmaceutique sous forme de gel selon l'une des revendications 1 à 29, pour une utilisation dans le traitement d'un trouble hormonodépendant chez un patient.

41. Préparation pharmaceutique sous forme de gel selon la revendication 40, **caractérisée en ce que** le trouble hormonodépendant est le cancer de la prostate, le cancer du sein, un myome de l'utérus, une endométriose ou une puberté précoce.

42. Préparation pharmaceutique sous forme de gel selon l'une des revendications 1 à 29, pour une utilisation dans la modification de la fonction reproductrice d'un patient.

43. Préparation pharmaceutique sous forme de gel selon l'une des revendications 40 à 42, **caractérisée en ce que** la préparation pharmaceutique sous forme de gel peut être administrée au patient par voie sous-cutanée ou intramusculaire.

44. Préparation pharmaceutique sous forme de gel selon l'une des revendications 40 à 42, **caractérisée en ce que** la préparation pharmaceutique sous forme de gel administrée présente une activité pharmaceutique prolongée.

45. Préparation pharmaceutique sous forme de gel selon la revendication 44, **caractérisée en ce que** la préparation pharmaceutique sous forme de gel administrée présente une activité pharmaceutique prolongée pendant au moins 4 semaines.

46. Préparation pharmaceutique sous forme de gel selon la revendication 44, **caractérisée en ce que** la préparation pharmaceutique sous forme de gel administrée présente une activité pharmaceutique prolongée pendant au moins 8 semaines.

47. Préparation pharmaceutique sous forme de gel selon la revendication 44, **caractérisée en ce que** la préparation pharmaceutique sous forme de gel administrée présente une activité pharmaceutique prolongée pendant au moins 12 semaines.
